(19) Europäisches Patentamt
European Patent Office
Office européen des brevets

(11) Publication number: **0 103 447**
**B1**

(12) **EUROPEAN PATENT SPECIFICATION**

(45) Date of publication of patent specification: **13.08.86**

(51) Int. Cl.$^4$: **C 07 C 49/92,** C 07 C 45/77

(21) Application number: **83305119.6**

(22) Date of filing: **05.09.83**

(54) **Volatile cerium complexes and production thereof.**

(30) Priority: **15.09.82 US 418216**
**28.06.83 US 508602**

(43) Date of publication of application:
**21.03.84 Bulletin 84/12**

(45) Publication of the grant of the patent:
**13.08.86 Bulletin 86/33**

(84) Designated Contracting States:
**AT BE CH DE FR GB IT LI NL SE**

(56) References cited:
**US-A-4 176 918**

**INORGANIC CHEMISTRY, vol. 6, no. 6, June 1967, pages 1105-1107; C.S. SPRINGER Jr. et al.: "Rare earth chelates of 1,1,1,2,2,3,3-heptafluoro-7,7-dimethyl-4,6-octanedione"**

**CHEMICAL ABSTRACTS, vol. 70, 1969, pp. 409, no. 92728q, Columbus, Ohio, (US); R.BELCHER et al.: "Volatile complex chelates of rare earth and alkali metals"**

(73) Proprietor: **Corning Glass Works**
**Houghton Park**
**Corning New York 14831 (US)**

(72) Inventor: **Thompson, David Allen**
**1259 Stoneybrook Drive**
**Horeseheads New York (US)**

(74) Representative: **Kyle, Diana et al**
**ELKINGTON AND FIFE High Holborn House**
**52/54 High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 103 447 B1**

**Description**

This invention relates to volatile cerium complexes and to the production thereof; more particularly it relates to organometallic compounds specifically to β-diketonate complexes of cerium, which exhibits unusually high volatility and stability.

Volatile metal complexes are of interest for a variety of applications, including use as fuel additives, metal vapour sources for vapour phase reactions and gas transport reagents. A useful discussion of β-diketonate complexes and the uses thereof is provided by R. E. Sievers, *et al.*, Science, *201,* [4352], pages 217—223, (July 1978), wherein numerous references to the preparation and use of these complexes are cited.

β-diketonates of the rare earth or lanthanide series of elements of the Periodic Table correspond to the general formula: $Ln(AA')_3$ wherein Ln represents the metal element and AA' represents the diketonate ligand which forms the complex. Discussions of the synthesis and properties of the rare earth β-diketonate complexes usually do not treat the cerium complexes. This is due to the complexity arising because cerium has two stable oxidation states.

The trivalent paramagnetic complexes of $Ce^{III}$ with β-diketones, such as acetylacetone $[Ce(acac)_3]$, trifluoroacetalacetone $[Ce(tfa)_3]$ and hexafluoroacetylacetone $[Ce(hfa)_3]$ are frequently mentioned, but only a few tetravalent ($Ce^{IV}$) compounds have been reported. These include $Ce(acac)_4$, $Ce(tfa)_4$ and complexes of cerium with 2,2,6,6 - tetramethyl - 3,5 - heptane - dione (thd) and the aromatic diketone $(C_6H_5CO)_2CH$, the first two being made by the oxidative decomposition of trivalent complexes incorporating the same ligands in an inert solvent in flowing air or oxygen. In the case of $Ce(tfa)_4$, yields are poor even in the presence of excess quantities of the free β-diketone (Htfa).

Cerium complexes have also been formed with some of the 8-carbon β-diketones, including 6,6,7,7,8,8,8 - heptafluoro - 2,2 - dimethyl - 3,5 - octane - dione [Ce(fod) - 3] and 2,2,7 - trimethyl - 3,5 - octane - dione $[Ce(tod)_4]$. However, none of these cerium complexes have exhibited sufficient stability and volatility to be truly useful as a cerium metal vapour source for vapour phase reactions, designed to produce cerium-containing products.

It is therefore an object of the present invention to provide a cerium complex and a precursor useful in the production of that complex, which result in a stable compound exhibiting sufficient volatility for use as a vapour source of cerium.

The present invention relates to a $Ce^{4+}$ β-diketonate complex of improved stability and volatility, as well as a group of volatile $Ce^{3+}$ β-diketonate complexes useful as intermediates in the production of the pure $CE^{4+}$ complex and as volatile sources for mixtures of cerium and alkali metals.

The complexes according to the present invention are complexes of cerium with the fluorinated β-diketone 6,6,7,7,8,8,8 - heptafluoro - 2,2 - dimethyl - 3,5 - octane - dione (Hfod), a β-diketone corresponding to the following formula:

$$CH_3—C(CH_3)_2—\overset{\overset{\displaystyle O}{\|}}{C}—CH_2—\overset{\overset{\displaystyle O}{\|}}{C}—CF_2—CF_2—CF_3$$

Cerium β-diketonate complexes of this β-diketone are produced by the deprotonation of the diketone at the C4 position to produce $(fod)^-$ anions or ligands. Four of these ligands then combine with a cerium ion to form a complex wherein the metal is in 8-fold coordination with the oxygen atoms in the ligands. The formula of the $Ce^{4+}$ complex with this β-diketone is $Ce(fod)_4$. The formula of the $Ce^{3+}$ complexes, which may be characterised as alkali metal β-diketonate salts, is $M[Ce(fod)_4]$ wherein M represents an alkali metal selected from Li, Na, K, Rb and Cs. These salts may be used to produce pure $Ce(fod)_4$, by a process of oxidation or they may be used as sources for Ce-M mixtures in vapour deposition reactions to form metal or oxide products.

$Ce(fod)_4$ offers significant advantages over prior art β-diketonate complexes of cerium with respect to both thermal stability and volatility. It has a vapour pressure at least two orders of magnitude higher than that of the 3-complex with the same ligand, $Ce(fod)_3$. Also, it is stable against decomposition at temperatures sufficiently high to permit vapours of the compound to be efficiently generated at substantial partial pressures. Thus, the compound is believed to be superior to previous cerium compounds when used, for example, as a vapour source of cerium in a chemical vapour deposition reaction.

The present invention may be further understood by reference to the accompanying drawings wherein:

Fig. 1 illustrates a proton nuclear magnetic resonance spectrum for the β-diketonate complex $Ce(fod)_4$;

Fig. 2 illustrates a thermogravimetric analysis curve demonstrating the volatility of $Cd(fod)_4$; and

Fig. 3 illustrates a comparison between the vapour pressures of $Ce(fod)_4$ and $Na[Ce(fod)_4]$ with selected known β-diketonate complexes.

The preparation of pure cerium (fod) complexes in accordance with the present invention involves the deprotonation of the β-diketone in the presence of cerium (III) nitrate. The following Example describes a suitable method for preparing such a complex.

Example 1

A 68.2 g sample of the β-diketone 6,6,7,7,8,8,8 - heptafluoro - 2,2 - dimethyl - 3,5 - octane - dione (hfod) is added to 115 ml of 2M aqueous $NH_4OH$ resulting in a white precipitate which is separated and dissolved in a mixture of 200 ml of water and 200 ml of methanol. The resulting

solution is placed in an addition funnel and is added dropwise to a nitric acid solution of cerium nitrate, the latter solution being made by adding 25.0 g $Ce(NO)_3 \cdot 6H_2O$ to 60 ml of 1.4 M $HNO_3$. After addition of the water-methanol solution, 2M $NH_4OH$ is added to the reaction mixture to achieve and maintain a pH of 6.

The resulting mixture separates into a red oil phase and an aqueous phase. The phases are stirred under oxygen at room temperature to obtain complete oxidation of $Ce^{3+}$ to $Ce^{4+}$. This may be accomplished within about 24 hours. Thereafter, hexanes (200 ml) are added and the hexane layer containing the product is separated from the aqueous phase, filtered and evaporated to dryness in a rotary evaporator.

The red solid is redissolved in 300 ml of hexanes and 600 ml of ethanol is added. Crystals of red $Ce(fod)_4$ form after cooling of the solution.

Proton nuclear magnetic resonance and magnetic susceptibility measurement of the product indicates that the yield is substantially entirely $Ce(fod)_4$, with no evidence of $Ce(fod)^{-4}$. Fig. 1 of the accompanying drawings illustrates a proton nuclear magnetic resonance spectrum of this product in a $CCl_4$ solvent against a tetramethylsilane standard at 60 MHz, from which the absence of trivalent cerium compounds of (fod) may be inferred.

In an alternative preparation procedure, $Ce(fod)_4$ is produced by the oxidation of the intermediate $Na[Ce(fod)_4]$. This intermediate results when the deprotonation of the β-diketone is carried out using NaOH. An illustrative procedure is set out in the following Example.

Example 2

A methanolic solution of cerium nitrate is prepared by adding 110 g of $Ce(NO_3)_3 \cdot 6H_2O$ to 250 ml of methanol. A methanolic solution of Hfod is then prepared by adding 300 g of the β-diketone to 250 ml of methanol in an addition funnel. This latter solution is then added to the cerium nitrate solution with continuous stirring.

After this addition has been completed, 4M NaOH is added to the mixture dropwise with pH monitoring until a pH of from 7 to 8 is attained. 600 ml of water and 500 ml of hexanes are then added to the reaction mixture and a separation of layers occurs with concentration of the β-diketonate product in the hexane layer.

The hexane layer is separated and rotary-evaporated to dryness. The product is a crystalline mixture of $Na[Ce(fod)_4]$ and $Ce(fod)_4$, with the proportion of the latter compound depending upon the extent of oxidation which has been permitted to occur after adding NaOH. Separation of the $Ce(fod)_4$ from the $Na[Ce(fod)_4]$ may be effected by dissolving the product in hexane and separating the $Na[Ce(fod)_4]$ from the solution by adding ethanol, in which the solubility of the sodium complex is relatively high.

Oxidation of $Na[Ce(fod)_4]$ to $Ce(fod)_4$

To convert the sodium complex to $Ce(fod)_4$, a 255 g sample of $Na[Ce(fod)_4]$ is dissolved in 500 ml of hexane and 500 ml of distilled water. This mixture is then refluxed at low temperature for about 60 hours, while bubbling pure $O_2$ gas through the liquid. At the conclusion of this oxidation treatment, the solution is deep red in colour, with a tan solid present on the sides of the reaction container.

The solution is separated, filtered and rotary-evaporated to dryness, the product consisting predominantly of $Ce(fod)_4$ with some residual $Na[Ce(fod)_4]$ which is separated from the pure cerium product by partitioning in an ethanol/hexane mixture. The tan solid recovered from the oxidation step is identified by x-ray diffraction as $CeO_2$.

The β-diketonate products produced in accordance with Example 2 above are examined by differential scanning calorimetry to determine melting points. The results are given in Table 1 below

TABLE 1
DSC endothermic peaks

| Compound | Major | Minor |
|---|---|---|
| $Ce(fod)_4$ | 97°C | 84°C, 156°C |
| $Na[Ce(fod)_4]$ | 120°C | 65°C |

The infrared spectra of the two complexes are similar over the region from 4,000 to 400 $cm^{-1}$, but exhibit important differences in the C=O region, due to the difference in the oxidation state of cerium. The C=O (C=C) stretch frequencies are expected to shift to lower regions as the oxidation state of the metal increases. For $Na[Ce(fod)_4]$, these stretching frequencies are evidenced by a strong peak at 1630 $cm^{-1}$ with a weak shoulder at 1645 $cm^{-1}$. For $Ce(fod)_4$, strong peaks are observed at 1595 $cm^{-1}$ and 1610 $cm^{-1}$.

The preparation of other $M[Ce(fod)_4]$ alkali metal salts would follow that described in Example 2 above, but replacing NaOH with a base, such as LiOH or KOH, in the deprotonation step. In this way salts, such as $Li[Ce(fod)_4]$ and $K[Ce(fod)_4]$, may be prepared which could be converted to $Ce(fod)_4$ or used directly as sources of metal mixtures.

The very low melting point of $Ce(fod)_4$, e.g. about 97°C, is a particular advantage where the compound is to be used to supply cerium-containing vapours for a vapour phase reaction. Both $Ce(tod)_4$ and $Ce(tfa)_3$ exhibit significantly higher melting points, e.g. 134°C and 176°C, respectively.

The high volatility and stability of the $Ce(fod)_4$ complex when compared with other trivalent and tetravalent cerium β-diketonates, are particularly important. Fig. 2 of the accompanying drawings illustrates a thermogravimetric analysis curve for $Ce(fod)_4$ which suggests rapid and complete volatilization of the compound with no evidence of decomposition at temperatures below 275°C. The temperature of 50% volatization of the

sample ($T_{1/2}$), a useful relative measure of volatility, is about 250°C and no unusual weight fluctuations evidencing thermal decomposition during the course of vaporization are evident.

This volatilization behaviour is substantially better than that exhibited by any of Ce(tod)$_4$, Ce(hfa)$_3$ or Ce(tfa)$_3$. Ce(tod)$_4$ exhibits no evidence of decomposition, but has a $T_{1/2}$ temperature of about 320°C, while both Ce(tfa)$_3$ and Ce(hfa)$_3$ exhibit evidence of significant thermal decomposition at these volatilization temperatures during TGA analysis.

The high vapour pressure of the tetravalent cerium (fod) complex also favours its use as a source of cerium vapours for vapour phase reactions. Fig. 3 of the accompanying drawings plots vapour pressure as a function of temperature for the cerium 2-diketonate complexes Ce(fod)$_4$, Na[Ce(fod)$_4$], Ce(fod)$_3$, Ce(tfa)$_3$, and Ce(tod)$_4$. The data for the complex Ce(fod)$_3$ was taken from the literature. It is evident from a study of accompanying Fig. 3 that the complex Ce(fod)$_4$ exhibits significantly higher vapour pressure than any of the Ce(tod)$_4$, Ce(tfa)$_3$, and Ce(fod)$_3$ complexes, particularly at temperatures above 100°C where these complexes would be used as vapour sources for cerium metal.

Accompanying Fig. 3 also demonstrates the very significant vapour pressure of the Na[Ce(fod)$_4$] complex. Although analogous alkali metal salts of other rare earth metal diketonates have shown some volatility, the very high volatility of this complex is surprising, suggesting that it could be used as a source of Ce/Na-containing vapours for vapour deposition applications where such a combination is required. It is expected that the other alkali metal salts of this complex would also have this utility.

A particularly preferred method for making Ce(fod)$_4$ is based on the fact that this complex may be formed immediately and quantitatively by reacting the deprotonated diketone (fod$^-$) with a soluble cerium (IV) compound, such as cerium (IV) salt. Convenient sources of dissolved cerium (IV) salts are soluble inorganic cerate complexes. For example, cerium (IV) nitrate may be obtained from the $(NH_4)_2Ce(NO_3)_6$ complex ammonium hexanitrato cerate. The reaction of the dissolved salt with the deprotonated diketone immediately produces the desired Ce(fod)$_4$ complex and the product is essentially free of cerium (III) complexes, such as Ce(fod)$_4^-$.

Particular cerium (IV) salts which may be used to produce the Ce(fod)$_4$ complex in accordance with the present invention include the nitrate, sulphate and perchlorate salts of $Ce^{4+}$. These are not handled directly, but are instead produced by dissolving complex cerate salts or acids in aqueous acidic solutions, e.g. solutions of $HNO_3$ or other mineral acid.

Among the cerate complexes which could be used as cerium (IV) sources are $(NH_4)_2Ce(NO_3)_6$, $(NH_4)_4Ce(SO_4)_4 \cdot 2H_2O$, $H_4Ce(SO_4)_4$ and $H_2Ce(ClO_4)_6$. However, cerium (IV) nitrate solutions produced from the $(NH_4)_2Ce(NO_3)_6$ starting material are presently preferred because they are anhydrous salts and are available in substantially higher purity (being essentially free of iron or other rare earth elements) than the other cerium (IV) starting materials.

As illustrated by the following Example, this method avoids the oxidation step necessary to convert the cerium (III) complex and thus provides a more rapid and convenient synthesis.

Example 3

A 15.4 gram sample of ceric ammonium nitrate (ammonium hexanitrato cerate), $(NH_4)_2Ce(NO_3)_6$ is dissolved in 25 ml of 1.4 M nitric acid with vigorous stiring to form a ceric nitrate solution. A primary standard certified grade of the cerate, commercially available from the G. F. Smith Chemical Co., Colombus, Ohio, U.S.A. is used.

In a separate flask, 34.2 grams of 6,6,7,7,8,8,8 - heptafluoro - 2,2 - dimethyl - 3,5 - octane - dione (Hfod) is mixed with 75 ml of 2M $NH_4OH$ to give a white precipitate of $NH_4$fod. This white precipitate is dissolved in about 200 ml of methanol to form a ligand solution.

The thus-prepared ligand solution is slowly added to the ceric nitrate solution with stiring and a red complex of the desired Ce(fod)$_4$ product is immediately formed. After stirring for 2 hours at room temperature the product is separated from the reaction mixture by filtration and dried in a vacuum. The yield is quantitative. Additional purification by recrystallization from hexane-ethanol or sublimation is possible, if desired. The identity of the product is confirmed by $^1H$ nuclear magnetic resonance spectroscopy to be the desired Ce(fod)$_4$.

**Claims**

1. A complex characterised in that it is a β-diketonate complex of $Ce^{4+}$ with 6,6,7,7,8,8,8 - heptafluoro - 2,2 - dimethyl - 3,5 - octane - dione (hfod), which corresponds to the following formula:

$$Ce(fod)_4.$$

2. A complex characterised in that it is a β-diketonate complex of $Ce^{3+}$ and $M^+$ with 6,6,7,7,8,8,8 - heptafluoro - 2,2 - dimethyl - 3,5 - octane - dione (hfod) which corresponds to the following formula:

$$M[Ce(fod)_4]$$

wherein M represents Na, Li, K, Cs and Rb.

3. A complex as claimed in claim 2 wherein M represents Na.

4. A process for the preparation of tetrakis - (6,6,7,7,8,8,8 - heptafluoro - 2,2 - dimethyl - 3,5 - octane - dione) cerium (IV), [Ce(fod)$_4$], as claimed in claim 1 characterised in that it comprises reacting deprotonated 6,6,7,7,8,8,8 - heptafluoro - 2,2 - dimethyl - 3,5 - octane - dione with a soluble cerium (IV) salt.

5. A process as claimed in claim 4 wherein the cerium (IV) salt is cerium (IV) nitrate.

6. A process as claimed in claim 5 wherein the cerium (IV) nitrate is produced by dissolving $(NH_4)_2Ce(NO_3)_6$ in an acidic edium.

**Patentansprüche**

1. Komplex, dadurch gekennzeichnet, daß er ein β - Diketonat - Komplex von $Ce^{4+}$ mit 6,6,7,7,8,8,8 - Heptafluoro - 2,2 - dimethyl - 3,5 - octan - dion (hfod) ist, entsprechend der folgenden Formel:

$$Ce(fod)_4.$$

2. Komplex, dadurch gekennzeichnet, daß er ein β - Diketonat - Komplex von $Ce^{3+}$ und $M^+$ mit 6,6,7,7,8,8,8 - Heptafluoro - 2,2 - dimethyl - 3,5 - octan - dion (hfod) ist, welcher der folgenden Formel:

$$M[Ce(fod)\cdot_4]$$

entspricht, wobei M Na, Li, K, Cs und Rb bedeutet.

3. Komplex, nach Anspruch 2, worin M Na bedeutet.

4. Verfahren zur Herstellung von Tetrakis - (6,6,7,7,8,8,8 - heptafluoro - 2,2 - dimethyl - 3,5 - octan - dion) - Zer (IV), $[Ce(fod)_4]$, nach Anspruch 1, dadurch gekennzeichnet, daß entprot nisiertes 6,6,7,7,8,8,8 - Heptafluoro - 2,2 - dimethyl - 3,5 - octan - dion with einem löslichen Zer (IV)-Salz umgesetzt wird.

5. Verfahren nach Anspruch 4, wobei das Zer (IV)-Salz Zer (IV)-Nitrat ist.

6. Verfahren nach Anspruch 5, wobei das Zer (IV)-Nitrat durch Lösen von $(NH_4)_2Ce(NO_3)_6$ in einem sauren Medium hergestellt wird.

**Revendications**

1. Un complexe caractérisé en ce que c'est un complexe β-dicétonate de $Ce^{4+}$ avec la 6,6,7,7,8,8,8 - heptafluoro - 2,2 - diméthyl - 3,5 - octane dione (hfod), qui correspond à la formule suivante:

$$Ce(fod)_4.$$

2. Un complexe caractérisé en ce que c'est un complexe β-dicétonate de $Ce^{3+}$ et $M^+$ avec la 6,6,7,7,8,8,8 - heptafluoro - 2,2 - diméthyl - 3,5 - octane dione (hfod), qui correspondant à la formulae suivante:

$$M[Ce(fod)_4],$$

où M représente Na, Li, K, Cs et Rb.

3. Un complexe selon la revendication 2, caractérisé en ce que M représente Na.

4. Procédé pour la préparation du tétrakis - (6,6,7,7,8,8,8 - heptafluoro - 2,2 - diméthyl - 2,5 - octane dione cérium (IV), $[Ce(fod)_4]$, selon la revendication 1, caractérisé en ce qu'il comprend la mise en réaction de 6,6,7,7,8,8,8 - heptafluoro - 2,2 - diméthyl - 3,5 - octane dione déprotonée avec un sel soluble de cérium (IV).

5. Procédé selon la revendication 4, caractérisé en ce que le sel de cérium (IV) est le nitrate de cérium (IV).

6. Procédé selon la revendication 5, caractérisé en ce que le nitrate de cérium (IV) est obtenu par dissolution de $(NH_4)_2Ce(NO_3)_6$ dans un milieu acide.

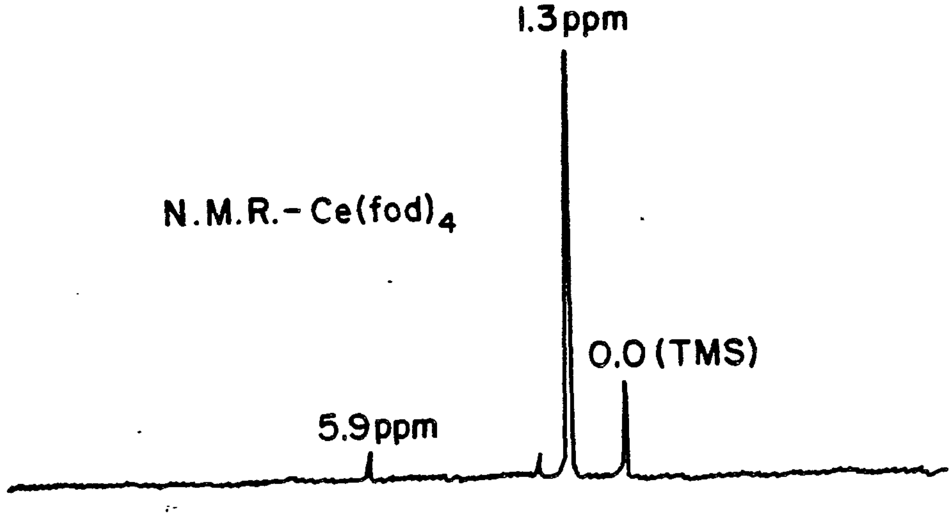
1.3ppm
N.M.R.- Ce(fod)4
0.0 (TMS)
5.9ppm

*Fig. 1*

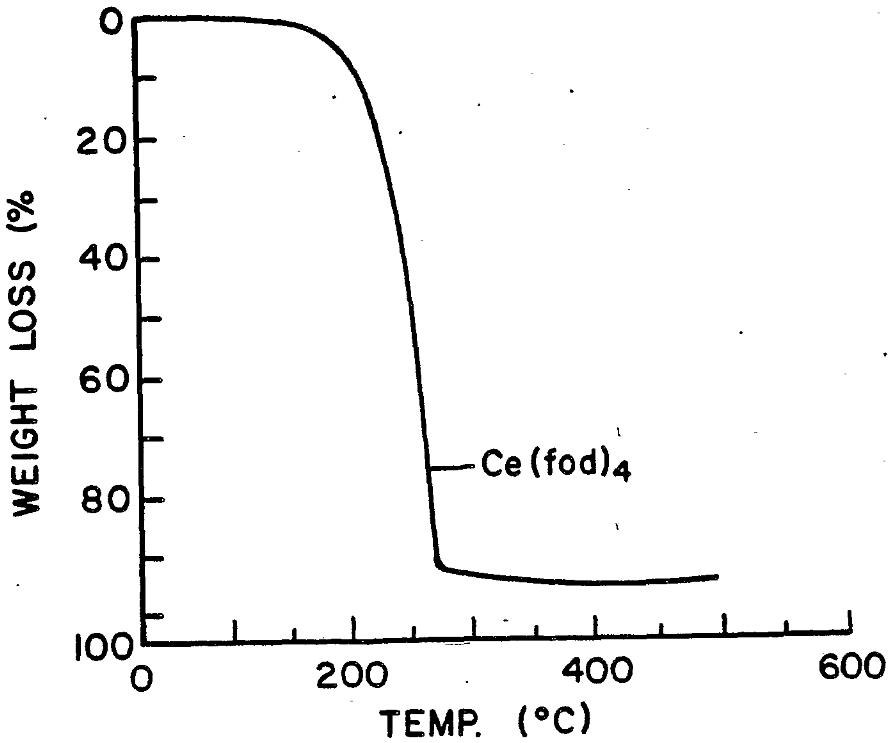
WEIGHT LOSS (%
0
20
40
60
80
100
0
200
400
600
TEMP. (°C)
Ce (fod)4

*Fig. 2*

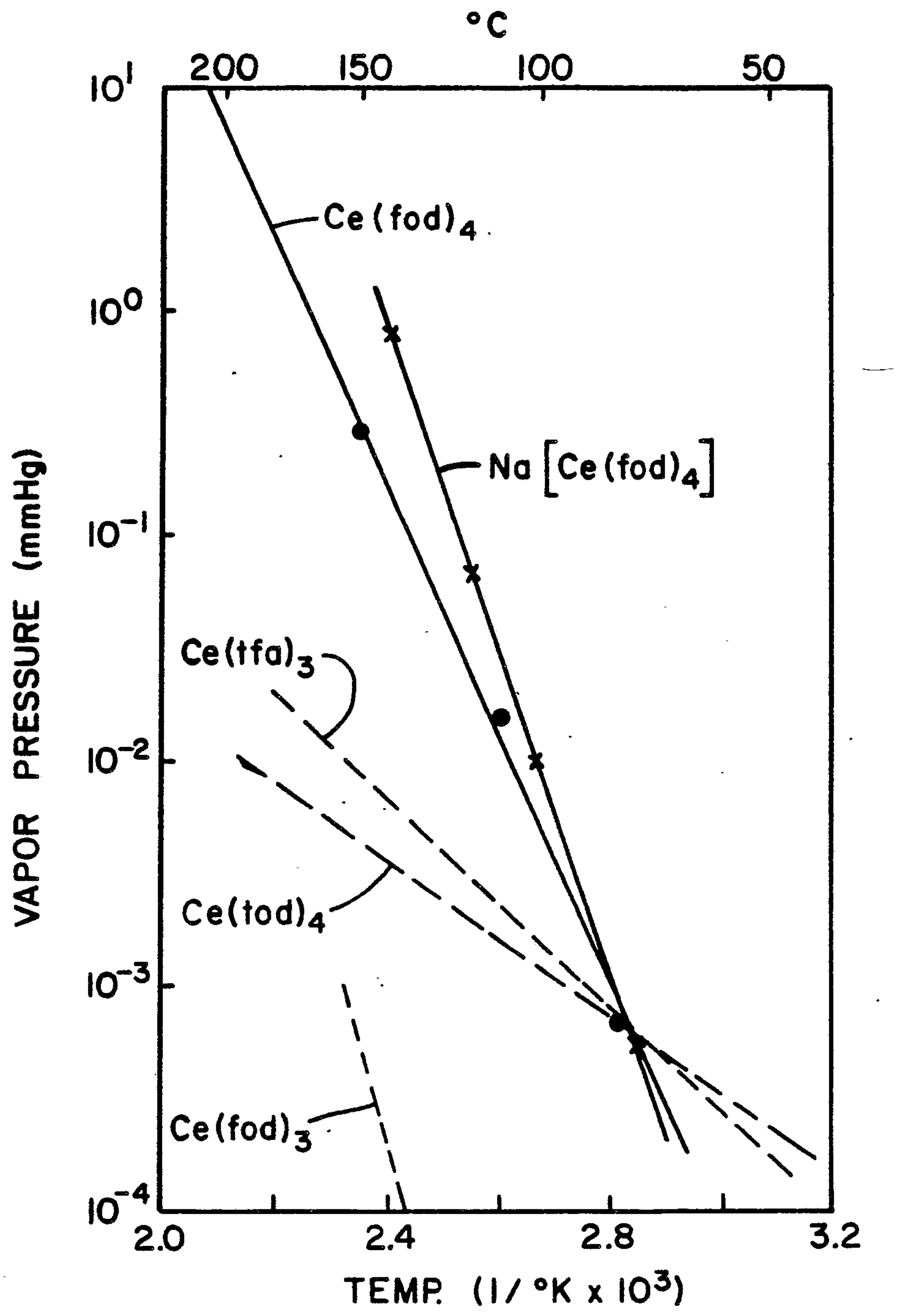
°C
200
150
100
50
$10^1$
$10^0$
$10^{-1}$
$10^{-2}$
$10^{-3}$
$10^{-4}$
VAPOR PRESSURE (mmHg)
Ce(fod)4
Na [Ce(fod)4]
Ce(tfa)3
Ce(tod)4
Ce(fod)3
2.0
2.4
2.8
3.2
TEMP. (1/°K x $10^3$)

*Fig. 3*